# EUROPÄISCHE PATENTSCHRIFT

(11) **EP 1 071 420 B1**
(45) Veröffentlichungstag und Bekanntmachung des Hinweises auf die Patenterteilung: **14.09.2005**
(21) Anmeldenummer: 99910372.4
(22) Anmeldetag: 22.03.1999
(51) Int. Cl.: A61K 31/40, A61P 35/00

(54) **INDOLYL-3-GLYOXYLSÄURE-DERIVATE MIT ANTITUMORWIRKUNG**
INDOLYL-3-GLYOXYLIC ACID DERIVATIVES WITH ANTITUMORAL ACTIVITY
DERIVES D'ACIDE INDOLYL-3-GLYOXYLIQUE A ACTION ANTITUMORALE

(30) Priorität: 02.04.1998 DE 19814838
(43) Veröffentlichungstag der Anmeldung: 31.01.2001
(73) Patentinhaber: Baxter Healthcare S.A., 8304 Wallisellen (CH); Baxter International Inc., Deerfield, IL 60015 (US)
(72) Erfinder: NICKEL, Bernd, D-64367 Mühltal (DE); SZELENYI, Istvan, D-90571 Schwaig (DE); SCHMIDT, Jürgen, D-88690 Uhldingen-Mühlhofen (DE); EMIG, Peter, D-63486 Bruchköbel (DE); REICHERT, Dietmar, D-63863 Eschau (DE); GÜNTHER, Eckhard, D-63477 Maintal (DE); BRUNE, Kay, D-91080 Marloffstein (DE)
(74) Vertreter: Müller-Boré & Partner Patentanwälte
(86) Internationale Anmeldenummer: PCT/EP1999/001918
(87) Internationale Veröffentlichungsnummer: WO 1999/051224

(56) Entgegenhaltungen:
- DE-A- 19 636 150

## Beschreibung

Indol-3-glyoxylamide finden als pharmakodynamisch aktive Verbindungen und als Synthesebausteine in der pharmazeutischen Chemie eine vielfältige Verwendung.

In der Patentanmeldung Neth. Appl. 6502481 sind Verbindungen beschrieben, die über ein antünflammatorisches und antipyretisches Wirkprofil und analgetische Aktivität verfügen.

In der britischen Anmeldung GB-PS 1 028 812 finden Derivate der Indolyl-3-glyoxylsäure und deren Amide Erwähnung als analgetisch, antikonsulsiv und ß-adrenergisch wirksame Verbindungen.

G. Domschke et al. (Ber. 94, 2353 (1961)) beschreibt 3-Indolyl-glyoxylamide, die pharmakologisch nicht charakterisiert sind.

E. Walton berichtet in J.Med.Chem. 11,1252 (1968) über Indolyl-3-glyoxylsäure-Derivate, die inhibitorisch auf die Glycerophosphat-Dehydrogenase und Lactat-Dehydrogenase wirken.

In der Europäischen Patentschrift EP 675110 werden 1H-Indol-3-glyoxylsäureamide beschrieben, die als sPLA2-lnhibitoren profiliert werden und bei Behandlung des septischen Schocks, bei Pankreatitis, bei der Behandlung allergischer Rhinitis und rheumatischer Arthritis zur Anwendung kommen.

Ziel der vorliegenden Erfindung ist es, N-substituierte Indol-3-glyoxylamide zur Verfügung zu stellen, die eine Antitumor-Wirkung besitzen und somit den verfügbaren Arzneischatz zu bereichern.
Die genannten Verbindungen sind bereits als Arzneimittel mit antiasthmathischer, antiaUergischer und immunsuppressiver/immunmodulierender Wirkung aus DE- OS 196 36 150 A1 bekannt.

Der Gegenstand der Erfindung umfaßt daher die Verwendung von N-substituierten Indol-3-gloxylamiden der allgemeinen Formel 1 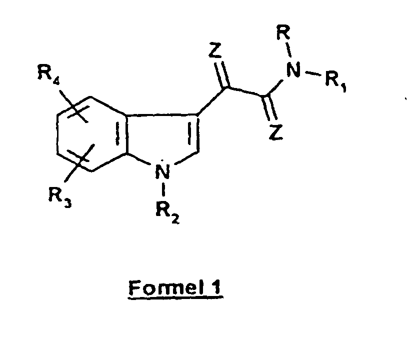 wobei die Reste R, R₁, R₂, R₃, R₄ und Z folgende Bedeutung haben:
R = Wasserstoff
R₁ = 4-Pyridyl, 4-Fluorophenyl
R₂ = Benzyl, 4-Chlorbenzyl, 4-Fluorbenzyl, 3-Pyridylmethyl, 4-Brombenzyl
R₃ und R₄ = Wasserstoff und
Z = Sauerstoff,
sowie deren physiologisch verträgliche Säureadditionssalze bzw. sofern möglich deren N-Oxide, zur Herstellung eines Arzneimittels zur Behandlung von Tumorerkrankungen.

Die Verbindungen können auch als Säureadditionssalze eingesetzt werden, beispielsweise als Salze von Mineralsäuren, wie beispielsweise Salzsäure, Schwefelsäure, Phosphorsäure, Salze von organischen Säuren, wie beispielsweise Essigsäure, Milchsäure, Malonsäure, Maleinsäure, Fumarsäure, Gluconsäure, Glucuronsäure, Zitronensäure, Embonsäure, Methansulfonsäure, Trifluoressigsäure, Bemsteinsäure und 2-Hydroxyethansulfonsäure.
Sowohl die Verbindungen der Formel 1 als auch deren Salze sind biologisch aktiv.
Die Verbindungen der Formel 1 können in freier Form oder als Salze mit physiologisch verträglichen Säuren verabreicht werden.
Die Applikation kann perorat, parenteral, intravenös, transdermal oder inhalativ vorgenommen werden.
Weiterhin betrifft die Erfindung pharmazeutische Zubereitungen mit einem Gehalt an mindestens einer der Verbindungen der Formel 1 oder deren Salze mit physiologisch verträglichen anorganischen oder organischen Säuren und gegebenenfalls pharmazeutisch verwendbaren Träger- und/oder Verdünnungs - bzw. Hilfsstoffen.

Als Applikationsformen eignen sich beispielsweise Tabletten, Dragees, Kapseln. Lösungen zur Infusion oder Ampullen, Suppositorien, Pflaster, inhalativ einsetzbare Pulverzubereitungen, Suspensionen ,Cremes und Salben.

Die Verfahren zur Herstellung der erfindungsgemäßen Verbindungen werden in den folgenden Reaktionsschemata 1 und 2 sowie in allgemeinen Vorschriften beschrieben. Alle Verbindungen lassen sich, wie beschrieben, oder analog herstellen.

Die Verbindungen der allgemeinen Formel 1 mit Z=O, R₁ = Aryl, Aralkyl, Heteroaryl und Heteroaralkyl sowie R₂= Alkyl, Aralkyl und Heteroaralkyl sind gemäß des folgenden Schemas 1 erhältlich:

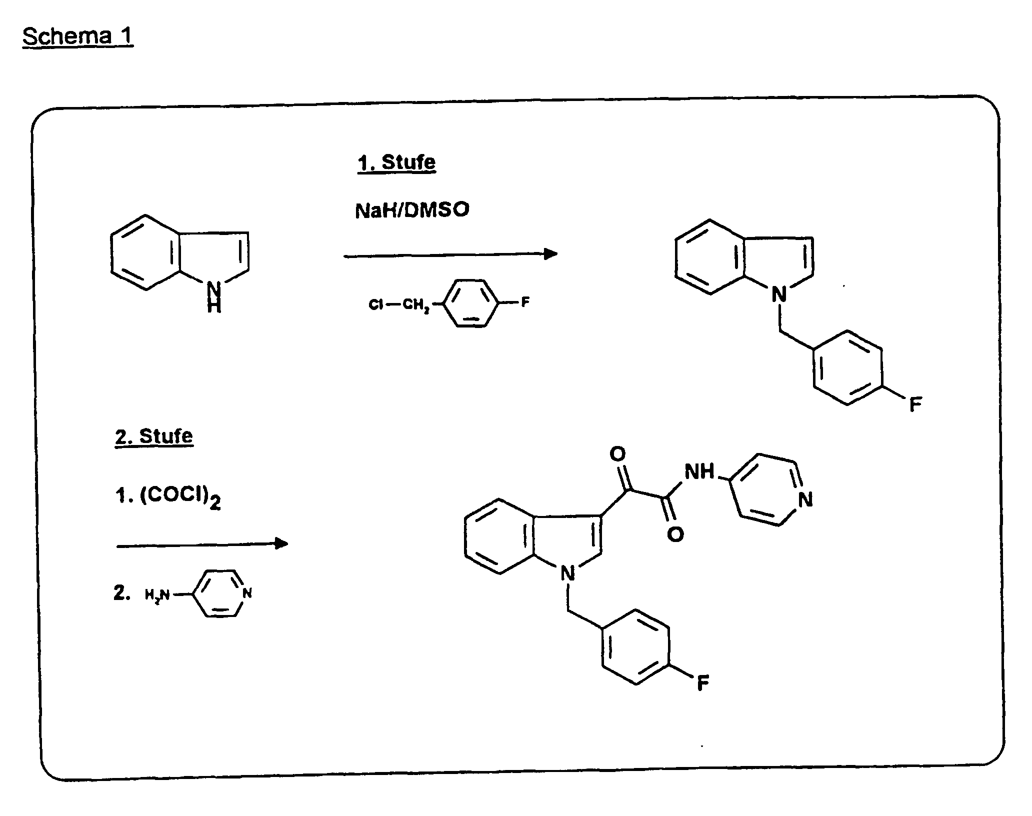

### 1.Stufe:

Das Indol-Derivat, das unsubstituiert oder an C-2 oder im Phenylgerüst einfach oder mehrfach substituiert sein kann, wird in einem protischen, dipolar aprotischen oder unpolaren organischen Lösungsmittel, wie beispielsweise Isopropanol, Tetrahydrofuran, Dimethylsulfoxid, Dimethylformamid, Dimethylacetamid, N-Methylpyrrolidon, Dioxan, Toluol oder Methylenchlorid gelöst und tropfenweise zu einer in einem Dreihalskolben unter N₂-Atmosphäre vorbereiteten molaren oder überschüssig eingesetzten Suspension einer Base, wie beispielsweise Natriumhydrid, pulversiertes Kaliumhydroxid, Kalium-tert.-butylat, Dimethylaminopyridin oder Natriumamid in einem geeigneten Lösungsmittel gegeben. Sodann gibt man beispielsweise das gewünschte Alkyl-, Aralkyl-bzw. Heteroaralkylhalogenid gegebenenfalls unter Zusatz eines Katalysators, wie z.B. Kupfer, zu und läßt einige Zeit, beispielsweise 30 Minuten bis 12 Stunden, nachreagieren und hält die Temperatur innerhalb eines Bereichs von 0°C bis 120°C, vorzugsweise zwischen 30°C bis 80°C, besonders zwischen 50°C und 65°C. Nach Beendigung der Reaktion wird die Reaktionsmischung in Wasser gegeben, die Lösung z.B. mit Diethylether, Dichlormethan, Chloroform, Methyl-tert.-butylether oder Tetrahydrofuran extrahiert und die jeweils erhaltene organische Phase mit wasserfreiem Natriumsulfat getrocknet. Man engt die organische Phase im Vakuum ein, kristallisiert den verbleibenden Rückstand durch Anreiben oder reinigt den öligen Rückstand durch Umkristallisation, Destillation oder durch Säulen- bzw. Flash-Chromatographie an Kieselgel oder Aluminiumoxid. Als Laufmittel dient beispielsweise ein Gemisch aus Dichlormethan und Diethylether im Verhältnis 8:2 (Vol/Vol) oder ein Gemisch aus Dichlormethan und Ethanol im Verhältnis 9:1 (Vol/Vol).

### 2.Stufe

Das nach obenstehender Vorschrift der 1. Stufe erhaltene N-substituierte Indol wird unter Stickstoffatmosphäre in einem aprotischen oder unpolaren organischen Lösungsmittel, wie beispielsweise Diethylether, Methyl-tert.-butylether, Tetrahydrofuran, Dioxan, Toluol, Xylol, Methylenchlorid oder Chloroform gelöst und zu einer unter Stickstoff-Atmosphäre bereiteten Lösung einer einfach molaren bis zu 60-prozentig überschüssigen Menge Oxalylchlorid in einem aprotischen oder unpolaren Lösungsmittel, wie z.B. in Diethylether, Methyl-tert.-butylether, Tetrahydrofuran. Dioxan, Toluol, Xylol, Methylenchlorid gegeben, wobei die Temperatur zwischen -5°C und 20°C gehalten wird. Man erhitzt sodann die Reaktionslösung bei einer Temperatur zwischen 10°C und 130°C, vorzugsweise zwischen 20°C und 80°C, besonders zwischen 30°C und 50°C für einen Zeitraum von 30 Minuten bis zu 5 Stunden und dampft anschließend das Lösungsmittel ab. Der verbleibende Rückstand des auf diese Weise gebildeten "Indolyl-3-glyoxylsäurechlorids wird in einem aprotischen Lösungsmittel wie z.B. Tetrahydrofuran, Dioxan, Diethylether, Toluol oder auch in einem dipolar aprotischen Lösungsmittel, wie z.B. Dimethylformamid, Dimethylacetamid oder Dimethylsulfoxid gelöst, auf eine Temperatur zwischen 10°C und -15°C, vorzugsweise zwischen -5°C und 0°C, gekühlt und in Gegenwart eines Säurefängers mit einer Lösung des primären oder sekundären Amins in einem Verdünnungsmittel versetzt. Als Verdünnungsmittel kommen die oben zur Auflösung des Indolyl-3-glyoxylsäurechlorids verwendeten Lösungsmittel in Frage. Als Säurefänger finden Triethylamin, Pyridin, Dimethylaminopyridin, bas. tonenaustauscher, Natriumcarbonat, Kaliumcarbonat, pulverisiertes Kaliumhydroxid sowie überschüssiges, zur Reaktion eingesetztes, primäres oder sekundäres Amin Verwendung. Die Reaktion findet bei einer Temperatur von 0°C bis 120°C, vorzugsweise bei 20-80°C, besonders zwischen 40°C und 60°C statt. Nach 1-3 stündiger Reaktionszeit und 24-stündigem Stehen bei Raumtemperatur wird das Hydrochlorid des Säurefängers filtriert, das Filtrat i.Vak. eingeengt und der Rückstand aus einem organischen Lösungsmittel umkristallisiert oder durch Säulenchromatographie über Kieselgel oder Aluminiumoxid gereinigt. Als Laufmittel findet z.B. ein Gemisch aus Dichlormethan und Ethanol (95:5, Vol/Vol) Verwendung.

### Ausführungsbeispiele

Gemäß dieser allgemeinen Vorschrift für die Stufen 1 und 2 , denen das Syntheseschema 1 zugrundeliegt, wurden folgende Verbindungen synthetisiert, die unter Angabe der jeweiligen chemischen Bezeichnung aus der nachfolgenden Übersicht hervorgehen. In den Tabellen 1a - j auf den Seiten A - J sind aus der allgemeinen Formel 1 und den Substituenten R₁ -R₄ und Z die Stukturen dieser Verbindungen und ihre Schmelzpunkte zu ersehen:

### Beispiel 1

### N-(Pyridin-4-yl)-[1-(4-fluorbenzyl)-indol-3-yl] glyoxylamid (D 24241)

### 1. Stufe

### 1-(4-Fluorbenzyl)-indol

In eineMischung von 2,64g Natriumhydrid (0,11 Mol, Mineralölsuspension) in 100 ml Dimethylsulfoxid wird eine Lösung von 11,72g (0,1 Mol) Indol in 50 ml Dimethylsulfoxid gegeben. Man erhitzt 1,5 Stunden auf 60°C, läßt danach abkühlen und tropft 15,9g (0,11 Mol) 4-Fluorbenzylchlorid zu. Die Lösung wird auf 60°C erwärmt, über Nacht stehengetassen und sodann unter Rühren in 400 ml Wasser gegossen. Man extrahiert mehrmals mit insgesamt 150 ml Methylenchlorid, trocknet die organische Phase mit wasserfreiem Natriumsulfat, filtriert und engt das Filtrat i.Vak. ein. Der Rückstand wird i. Hochvakuum destilliert: 21,0g (96%d.Th.) Sdp. (0,5mm): 140°C

### 2. Stufe

### N-(Pyridin-4-yl)-[1-(4-fluorbenzyl)-indol-3-yl] glyoxylamid (D 24241)

Zu einer Lösung von 2,25 ml Oxalylchlorid in 25 ml Ether wird bei 0°C und unter N₂ tropfenweise eine Lösung von 4,75 g (21,1 mmol) 1-(4-Fluorbenzyl)-indol in 25 ml Ether gegeben. Man erhitzt 2 Stunden zum Rückfluß und dampft anschließend das Lösungsmittel ab. Sodann wurden zum Rückstand 50 ml Tetrahydrofuran zugefügt, die Lösung auf -5°C abgekühlt und tropfenweise mit einer Lösung von 4,66 g (49,5 mMol) 4-Aminopyridin in 200 ml THF versetzt. Man erhitzt 3 Stunden zum Rückfluß und läßt über Nacht bei Raumtemperatur stehen. Das 4-Aminopyridin Hydrochlorid wird abgesaugt, der Niederschlag mit THF gewaschen, das Filtrat i. Vak.eingeengt und der Rückstand aus Essigester umkristallisiert.
Ausbeute: 7,09 g (90% d.Th.)
Schmelzpunkt: 225-226°C

| Elementaranalyse: | | | |
|---|---|---|---|
| ber. | C 70,77 | H 4,32 | N 11,25 |
| gef. | C 71,09 | H 4,36 | N 11,26 |

### Beispiel 2, D 24242 N-(Pyridin-4-yl)-(1-methyl-indot-3-yl) glyoxylamid

### Beispiel 3, D 24834 N-(Pyridin-3-yl)-[1-(4-fluorbenzyl)-indol-3-yl]-glyoxylamid

### Beispiel 4, D 24835 N-(Pyridin-3-yl)-(1-benzylindol-3-yl)-glyoxylamid

### Beispiel 5, D 24836 N-(Pyridin-3-yl)-[1-(2-chlorbenzyl)-indol-3-yl]-glyoxylamid

### Beispiel 6, D 24840 N-(4-Fluorphenyl)-[1-(4-fluorbenzyl)-indol-3-yl]-glyoxylamid

### Beispiel 7, D 24841 N-(4-Nitrophenyl)-[1-(4-fluorbenzyl)-indol-3-yl]-glyoxylamid

### Beispiel 8, D 24842 N-(2-Chlorpyridin-3-yl)-[1-(4-fluorbenzyl)-indol-3-yl]-glyoxylamid

### Beispiel 9, D 24843 N-(Pyridin-4-yl)-(1-benzylindol-3-yl)-glyoxylamid

### Beispiel 10, D 24848 N-(Pyridin-4-yl)-[1-(3-pyridylmethyl)-indol-3-yl]-glyoxylamid

### Beispiel 11, D 24849 N-(4-Fluorphenyl)-[1-(2-pyridylmethyl)-indol-3-yl]-glyoxylamid

### Beispiel 12, D 24850 N-(4-Fluorphenyl)-[1-(3-pyridylmethyl)-indol-3-yl]-glyoxylamid

### Beispiel 13, D 24851 N-(Pyridin-4-yl)-[1-(4-chlorbenzyl)-indol-3-yl]-glyoxylamid

### Beispiel 14, D 24852 N-(Pyridin-4-yl)-[1-(2-chlorbenzyl)-indot-3-yl]-glyoxylamid

### Beispiel 15, D 24853 N-(Pyridin-2-yl)-[1-(4-fluorbenzyl)-indol-3-yl]-glyoxylamid

### Beispiel 16, D 24847 N-(Pyridin-4-yl)-[1-(2-pyridylmethyl)-indol-3-yl]-glyoxylamid

### Beispiel 17, D 24858 (4-Phenyl-piperazin-1-yl)-[1-(4-fluorbenzyl)-indol-3-yl]-glyoxylamid

### Beispiel 18, D 24854 N-(Pyridin-2-yl)-(1-benzyl-indol-3-yl)-glyoxylamid

### Beispiel 19, D 25421 N-(Pyridin-4-yl)-[1-(4-fluorbenzyl)-6-ethoxycarbonylamino-indol-3-yl]-glyoxylamid

### Beispiel 20, D 25422N-(Pyridin-4-yl)-[1-(4-fluorbenzyl)-5-ethoxycarbonylamino-indol-3-yl]-glyoxylamid

### Beispiel 21, D 25423 N-(Pyridin-4-yl)-[1-(4-fluorbenzyl)-6-cyclopentyloxycarbonylamino-indol-3-yl]-glyoxylamid

### Beispiel 22, D 25420 4-(Pyridin-4-yl)-piperazin-1-yl)-[1-(4-fluorbenzyl)-indol-3-yl]-glyoxylamid

### Beispiel 23, D 24866 N-(3,4,5-Trimethoxybenzyl)-N-(allylaminocarbonyl-2-methyl-prop-1-yl)-[1-(4-fluorbenzyl)-indol-3-yl]-glyoxylamid

### Beispiel 24 N-(Pyridin-4-yl)-[1-(4-fluorbenzyl)-S-methoxy-indol-3-yl]-glyoxylamid

### Beispiel 25 N-(Pyridin-4-yl)-[1-(4-fluorbenzyl)-5-ethoxycarbonylamino-methylindol-3-yl]-glyoxylamid

### Ausgangsstufen für die nach Syntheseschema 1 hergestellten Verbindungen der allgemeinen Formel 1, die aus Tabelle 1 hervorgehen.

| | | | | |
|---|---|---|---|---|
| Für die Syntheseendstufen | D 24241 | D 24242 | D 24834 | D 24835 |
| | D 24836 | D 24840 | D 24841 | D 24842 |
| | D 24843 | D 24848 | D 24849 | D 24850 |
| | D 24851 | D 24852 | D 24853 | D 24847 |
| | D 24858 | D 24854 | D 25420 | D 25422 |
| | D 25421 | D 25423 sind alle Vorstufen käuflich. | | |

Weiterhin sind die Verbindungen der allgemeinen Formel 1 mit Z=O, R₁ = Aryl, Aralkyl, Heteroaryl, Heteroaralkyl und der Allylaminocarbonyl-2-methyl-prop-1-yl-Gruppe sowie R₂= alkyl,aralkyl und der Heteroaralkyl-Gruppe auch nach dem Syntheseweg des Schemas 2 erhältlich:

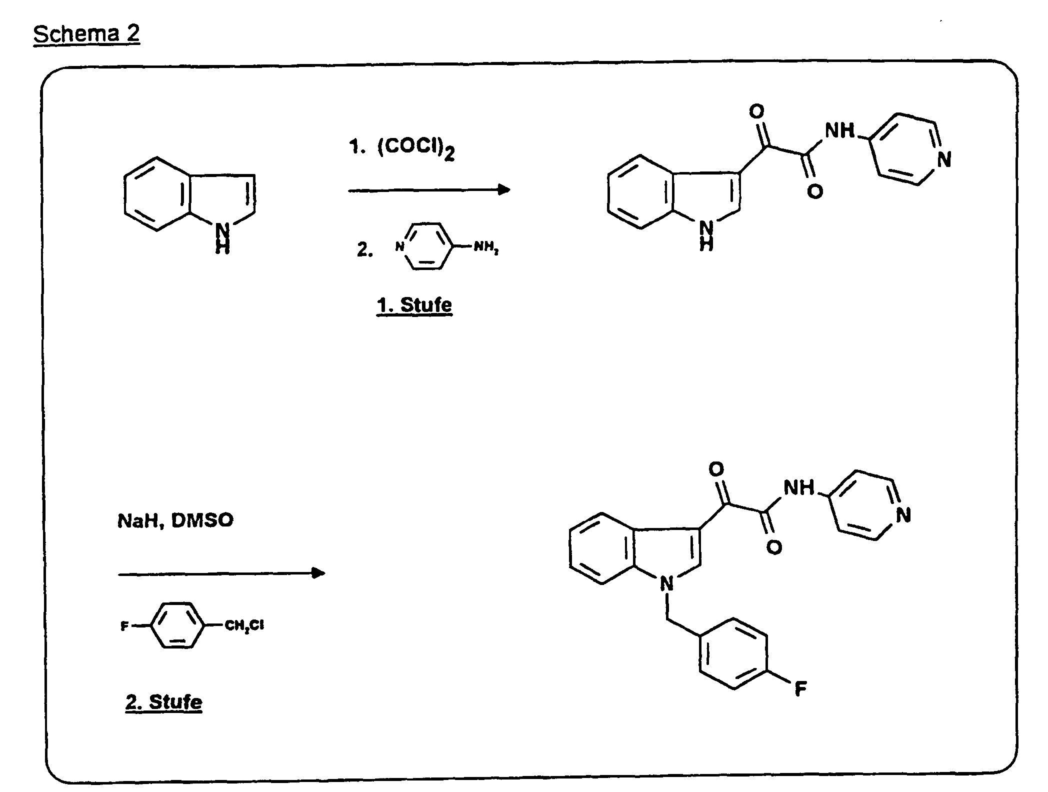

Nach dem vorliegenden Schema 2 wurden die Verbindungen D 24241, D24841, D 24840 und D 24834 (2. Stufe des Reaktionsschemas 2, s. auch Tabelle 1) sowie deren jeweilige Vorstufen D 24825, D 24831, D 24832 und D 24833 (1. Stufe des Reaktionsschemas 2, s. auch Tabelle 2 auf der Seite K) erhalten.

### N-(Pyridin-4-yl)-[1-(4-fluorbenzyl)-indol-3-yl]glyoxylamid (D 24241)

### 1. Stufe

### N-(Pyridin-4-yl)-(indol-3-yl)glyoxylamid

Zu einer Lösung von 9 ml Oxalylchlorid in 100 ml wasserfreiem Ether wird tropfenweise bei 0°C eine Lösung von 10 g (85.3 mmot) Indol in 100 ml Ether zugegeben. Man hält das Gemisch 3 Stunden unter Rückfluß. Sodann wird bei -5°C eine Suspension von 12 g (127,9 mMol) 4-Aminopyridin in 500 ml Tetrahydrofuran zugetropft, das Reaktionsgemisch unter Rühren 3 Stunden auf Rückflußtemperatur erhitzt und über Nacht bei Raumtemp. stehengelassen. Man filtrierte, behandelte den Niederschlag mit Wasser und reinigte die getrocknete Verbindung über eine Kieselgelsäule (Kieselgel 60, Fa. Merck AG, Darmstadt) unter Anwendung des Elutionsmittels Methylenchlorid/Ethanol (10:1.v/v).
Ausbeute: 9,8g (43,3% d.Th.)
Fp.: ab 250 °C

### 2. Stufe:

### N-(Pyridin-4-yl)-[1-[4-fluorbenzyl)-indol-3-yl]glyoxylamid (D24241)

Das nach der 1. Stufe erhaltene N-(Pyridin-4-yl)-(indol-3-yl)glyoxylamid wird gemäß der "Benzylierungsvorschrift" (Seite 5) mit 4-Fluorbenzylchlorid umgesetzt und die erhaltene Verbindung D 24241 isoliert.
Ausbeute: 41% d.Th.
Schmp.: 224-225°C

| | | | |
|---|---|---|---|
| Elementaranalyse | Ber. C 70,77 | H 4,32 | N 11,25 |
| | Gef. C 70,98 | H 4,40 | N 11,49 |

### Allgemeine Vorschrift zur Darstellung der Verbindungen der allgemeirlen Formel 1 gemäß Schema 2

### 1. Stufe:

Zu einer unter Stickstoffatmosphäre bereiteten Lösung einer einfach molaren bis zu 60% überschüssigen Menge Oxalylchlorid in einem aprotischen oder unpolaren Lösungsmittel, wie z.B. in Diethylether, Methyl-tert.-butylether, Tetrahydrofuran, Dioxan oder auch Dichlormethan, wird bei einer Temperatur zwischen -5°C und +5°C tropfenweise das in einem Lösungsmittel, wie z.B. oben für Oxalylchlorid angegeben, gelöste Indol-Derivat, das unsubstituiert oder an C-2 bzw. im Phenylring substituiert sein kann, zugegeben. Man erhitzt sodann die Reaktionslösung für 1 bis zu 5 Stunden auf eine Temperatur zwischen 10°C und 120°C, vorzugsweise zwischen 20°C und 80°C, besonders zwischen 30°C und 60°C und dampft anschließend das Lösungsmittel ab. Der verbleibende Rückstand des (Indol-3-yl) glyoxylsäurechlorids wird in einem aprotischen Lösungsmittel, wie z.B. Tetrahydrofuran, Dioxan, Diethylether, Toluol oder auch in einem dipolar aprotischen Lösungsmittel, wie z.B. Dimethylformamid, Dimethylacetamid oder Dimethylsulfoxid gelöst bzw. suspendiert, auf eine Temperatur zwischen -10°C und +10°C, vorzugsweise auf -5°C bis 0°C gekühlt und in Gegenwart eines Säurefängers mit einer Lösung des primären oder sekundären Amins in einem Verdünnungsmittel versetzt. Als Verdünnungsmittel kommen die zur Auflösung des "Indolyl-3-glyoxylsäurechlorids" verwendeten Lösungsmittel in Frage. Als Säurefänger finden Triethylamin, Pyridin, Dimethylaminopyridin, bas. lonenaustauscher, Natriumcarbonat, Kaliumcarbonat, pulverisiertes Kaliumhydroxid sowie überschüssiges, zur Reaktion eingesetztes primäres oder sekundäres Amin Verwendung. Die Reaktion findet bei einer Temperatur von 0°C bis 120°C, vorzugsweise bei 20-80°C, besonders zwischen 40°C und 60°C statt. Nach 1-4-stündiger Reaktionszeit und 24-stündigem Stehen bei Raumtemperatur wird filtriert,der Niederschlag mit Wasser digeriert, abgesaugt und i. Vak. getrocknet. Man reinigt die gewünschte Verbindung durch Umkristallisation in einem organischen Lösungsmittel oder durch Säulenchromatographie an Kieselgel oder Aluminiumoxid. Als Laufmittel findet z.B. ein Gemisch aus Dichlormethan und Ethanol (10:1, vol/vol) Verwendung.

### 2. Stufe

Das nach obenstehender Vorschrift der 1. Stufe erhaltene "Indol-3-yl-glyoxylamid" wird in einem protischen, dipolar aprotischen oder unpolaren organischen Lösungsmittel, wie z.B. in isopropanol, Tetrahydrofuran, Dimethylsulfoxid, Dimethylformamid, Dimethylacetamid, N-Methylpyrrolidon, Dioxan, Toluol oder Methylenchlorid gelöst und tropfenweise zu einer in einem Dreihalskolben unter N₂-Atmosphäre vorbereiten molaren oder überschüssig eingesetzten Suspension einer Base, wie z.B. Natriumhydrid, pulverisiertes Kaliumhydroxid, Kalium-tert-butylat, Dimethylaminopyridin oder Natriumamid in einem geeigneten Lösungsmittel gegeben. Sodann gibt man das gewünschte Alkyl-, Aralkyl-bzw. Heteroaralkylhalogenid entweder unverdünnt oder in einem Verdünnungsmittel, das z.B. auch zur Lösung des "Indol-3-yl-glyoxylamids" verwendet wurde, gegebenenfalls unter Zusatz eines Katalysators, wie z.B. Kupfer, zu und läßt einige Zeit, z.B. 30 Minuten bis 12 Stunden, reagieren und hält die Temperatur innerhalb eines Bereichs zwischen 0°C und 120°C, vorzugsweise zwischen 30°C und 80°C, besonders zwischen 50 und 70°C. Nach Beendigung der Reaktion wird das Reaktionsgemisch in Wasser gegeben, die Lösung z.B. mit Diethylether, Dichlormethan, Chloroform, Methyl-tert.-butylether, Tetrahydrofuran bzw. n-Butanol extrahiert und die jeweils erhaltene organische Phase mit wasserfreiem Natriumsulfat getrocknet.
Man engt die organische Phase im Vakuum ein, kristallisiert den verbleibenden Rückstand durch Anreiben bzw. reinigt den öligen Rückstand durch Destillation oder durch Säulen-bzw. Flashchromatographie an Kieselgel oder Aluminiumoxid. Als Laufmittel dient beispielsweise ein Gemisch aus Methylenchlorid und Diethylether im Verhältnis 8:2 (Vol/Vol) oder ein Gemisch aus Methylenchlorid und Ethanol im Verhältnis 9:1 (V/V)

Gemäß dieser allgemeinen Vorschrift für die Stufen 1 und 2, denen das Syntheseschema 2 zugrundeliegt, wurden die Verbindungen D 24241, D 24841, D 24840 und D 24834 synthetisiert, die auch schon gemäß des Sytheseablaufs des Reaktionsschemas 1 dargestellt wurden und aus Tabelle 1 hervorgehen. Die diesbezüglichen Vorstufen dieser Verbindungen sind aus Tabelle 2 auf Seite K und L ersichtlich.

Die Verbindungen zeigen eine gute dosisabhängige Antitumorwirkung in den folgenden pharmakologischen Modellen:

Die Indole besonders D-24851 und D-24241 sind zuerst im XTT- Proliferationstest-/Zytotoxizitätstest aufgefallen (Tab.3 und Tab. 3a). In diesem Testsystem wird der Einfluß von Substanzen auf das Proliferationsverhalten von Tumorzellinien untersucht. Dabei wird das zytotoxische Potential dieser Substanzen erfaßt. Die Testmethode ist bei Scudiero et al. 1988, Cancer Res. 48, 4827 beschrieben.

Es wurden bei den Untersuchungen folgende Tumorzellinien eingesetzt:
Die KB-Zellinie ein epidermales Karzinom der Mundhöhle,
die L1210-Zellinie eine lymphatische Leukämie der Maus,
die LNCAP-Zellinie ein Prostatakarzinom und
die SK-OV-3 Zeltinie ein Ovarial-karzinom.

In allen vier Tumorzeliinien war eine große Anzahl von verschiedenen Indolen wirksam. Die stärksten Wirkungen zeigten D-24851 und D-24241, wobei D-24851 wirksamer war als D-24241 (Tab.3 und 4).

in weiteren vergleichenden Untersuchungen mit D-24851 und D-24241 im Hohlfaser Assay an der Nacktmaus und an der L1210 (Maus) konnte bei beiden Verbindungen eine starke dosisabhängige Antitumorwirkung beobachte werden (Tab. 3 und 5). Im Hohlfaser Assay waren beide Verbindungen nahezu gleich stark wirksam, während an der L1210 D-24851 nach peroraler und intraperitonealer Gabe deutlich stärker wirksam war als D-24241. Im Vergleich zu den auf dem Markt befindlichen Antitumor-Substanzen ist D-24851 im Leukämiemodelt in vielen Fällen deutlich stärker wirksam als die bekannten Vergleichsubstanzen (Tab. 5).

Ein weiterer großer Vorteil von D-24851 im Vergleich zu den auf dem Markt befindlichen Antitumor-Substanzen ist die geringe Toxizität der Verbindung (Tab. 3 und 5). Mit LD 50 Werten von 1000 mg/kg p.o. und > 1000 mg/kg i.p. verfügt die Verbindung über eine große therapeutische Breite.
Weiterhin konnte nach Gabe von D-24851 keine DNA-Fragmentierung beobachtet werden. Auch im Haematopoese Versuch wurden keine der untersuchten Blutparameter durch die intraperitoneale Gabe von D-24851 verändert.

In einem weiteren Chemotherapiemodell dem Dunning -Tumor an der Ratte konnte nach mehrmaliger peroraler Gabe von D-24851 ein Tumorwachstumsstop und bei einigen Tieren sogar eine Tumorregression beobachtet werden.

Im KB-Versuch an der Nacktmaus konnte ebenfalls nach Gabe der beiden Indole D-24851 und D-24241 eine Antitumorwirkung beobachtet werden (Tab. 3, 3a und 4).

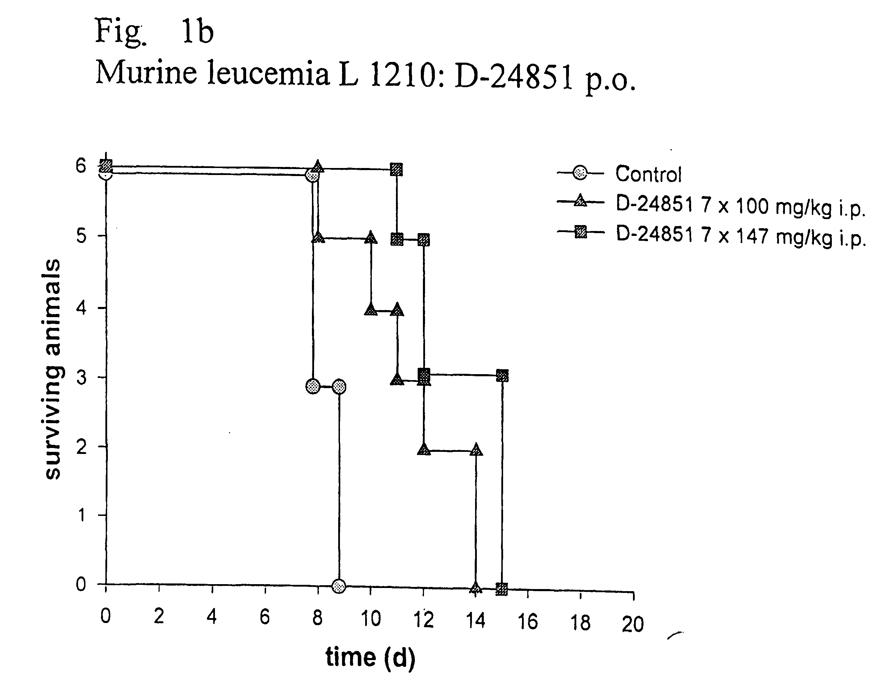

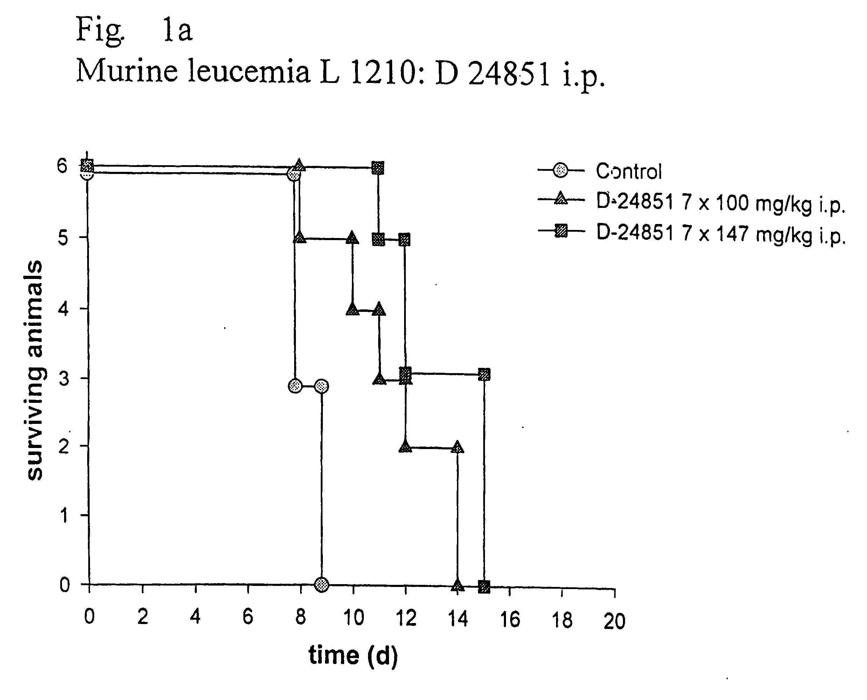

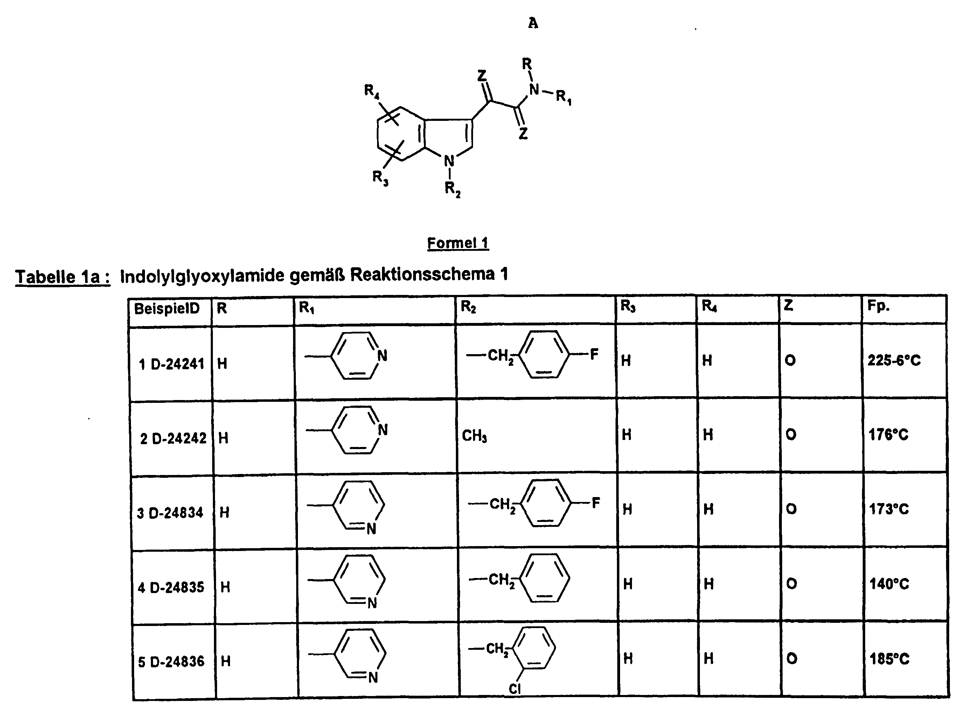

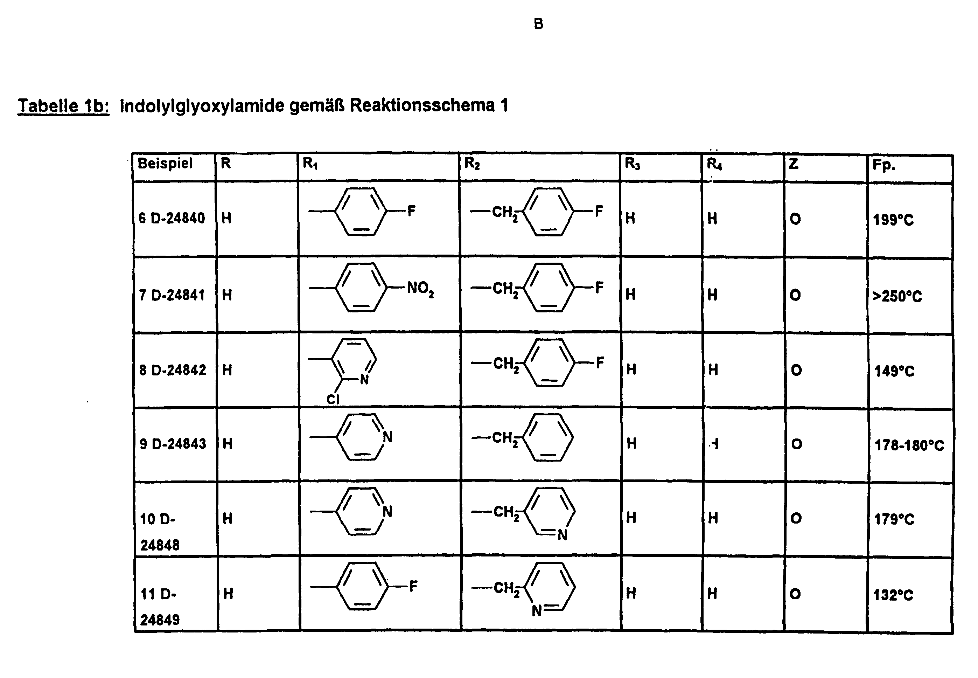

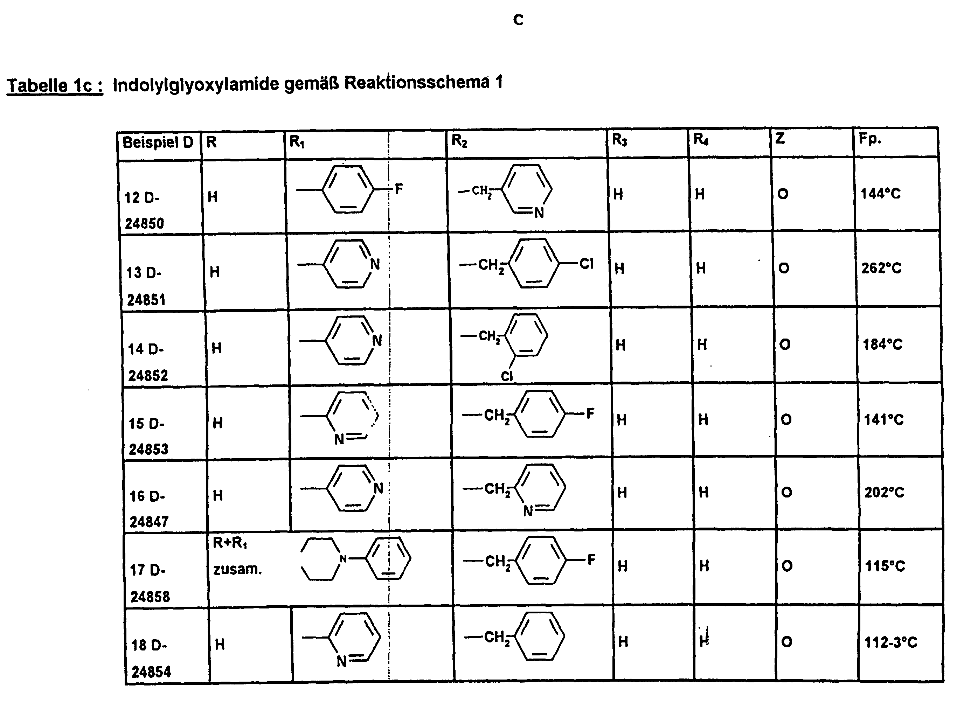

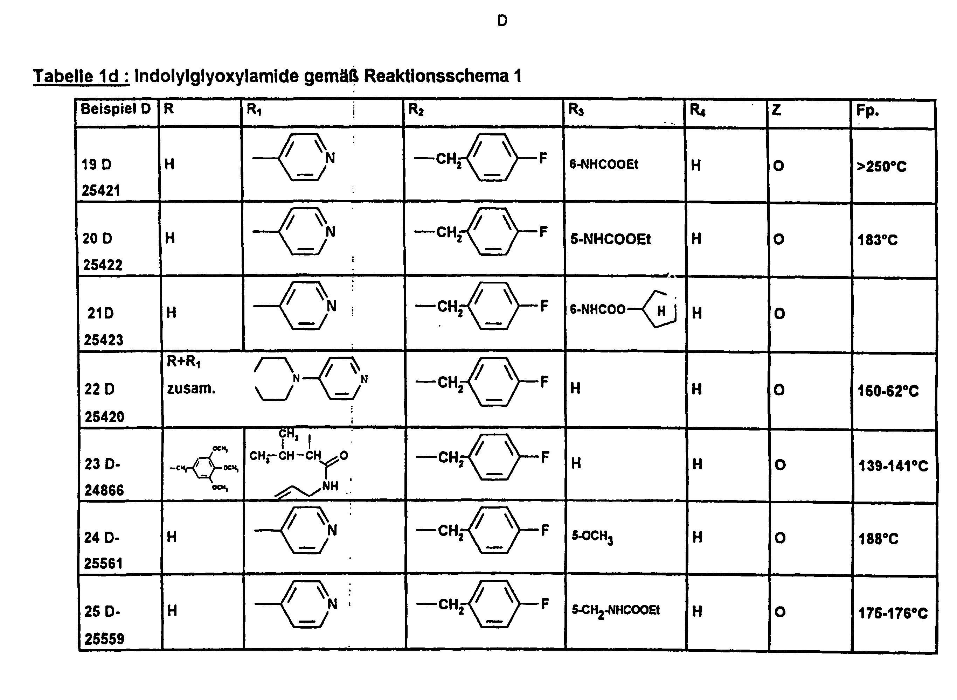

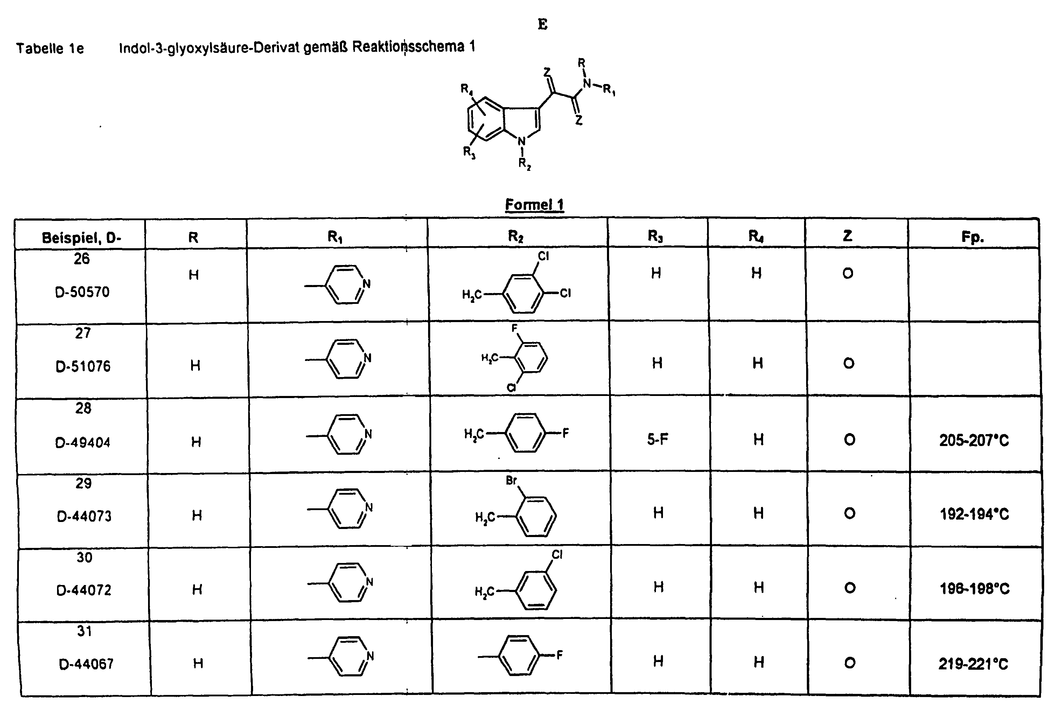

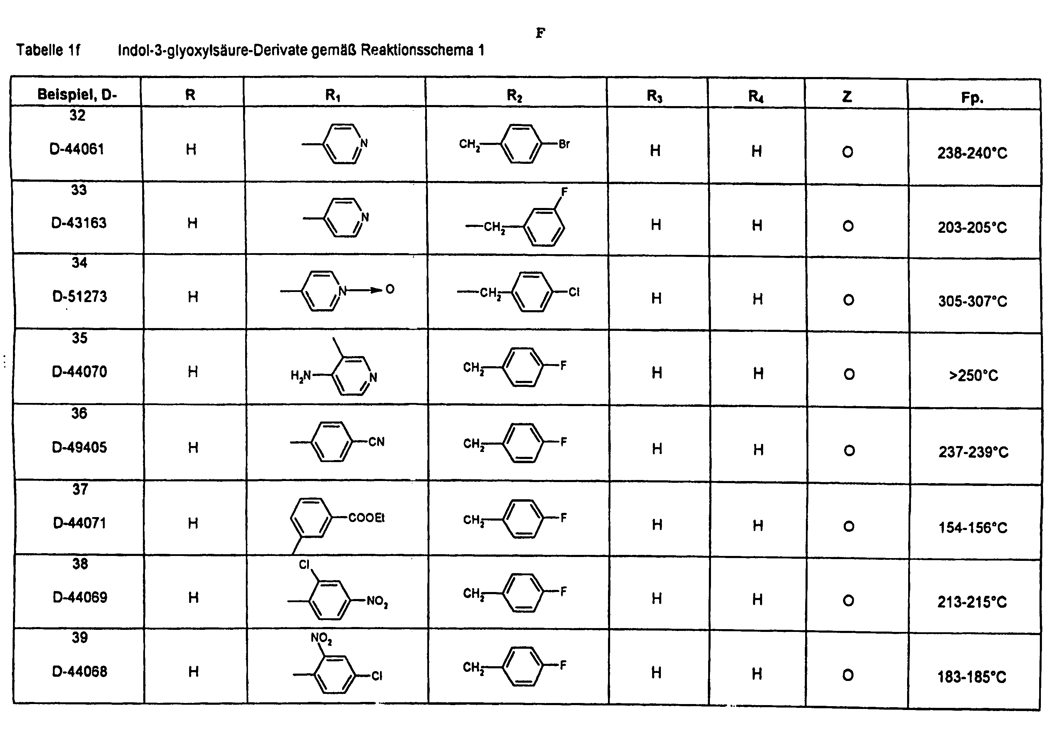

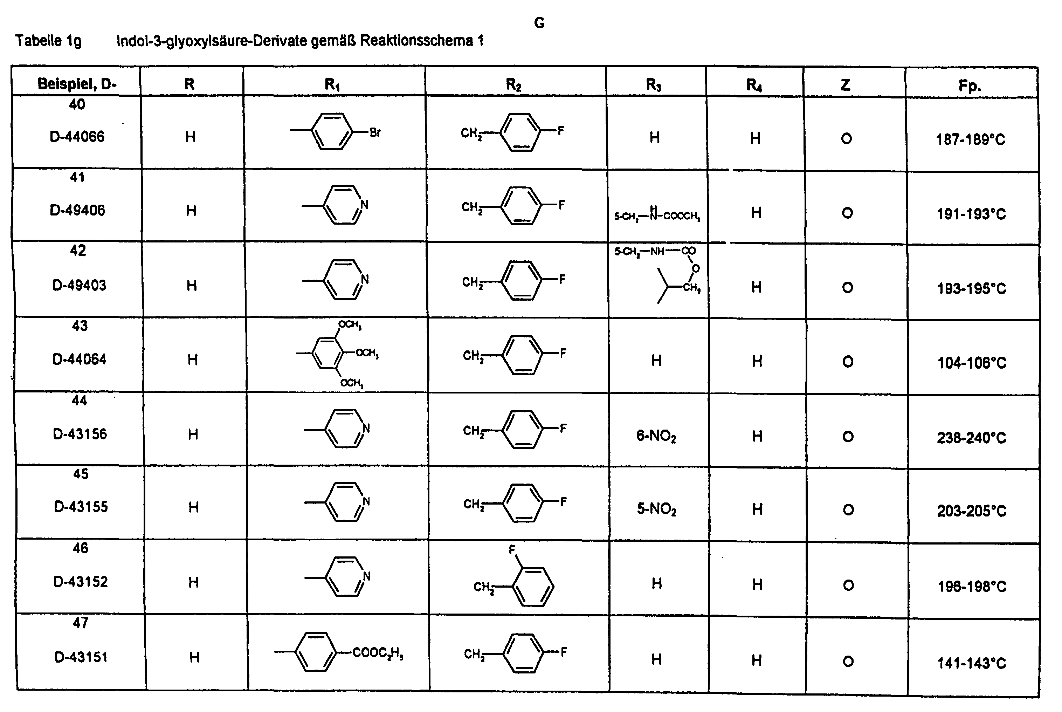

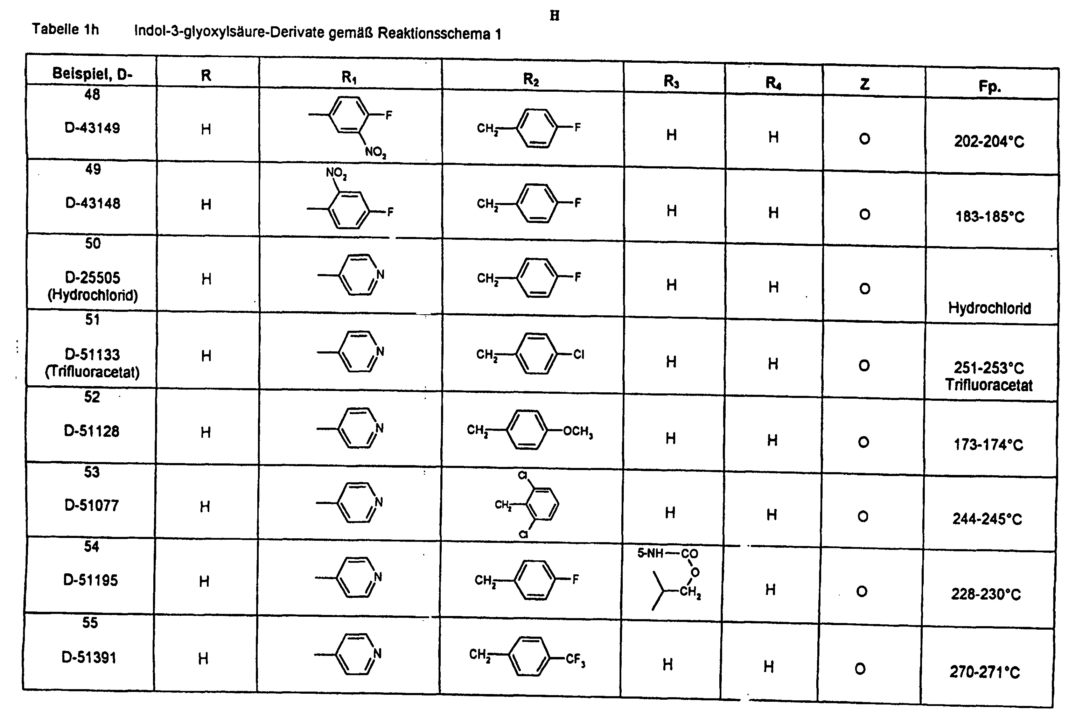

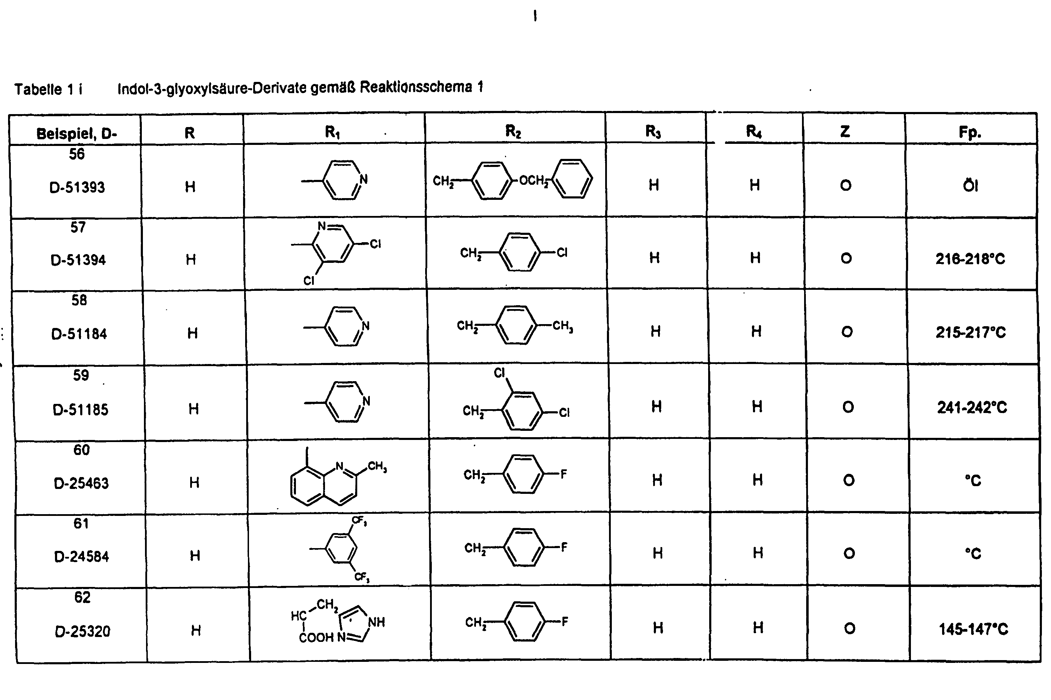

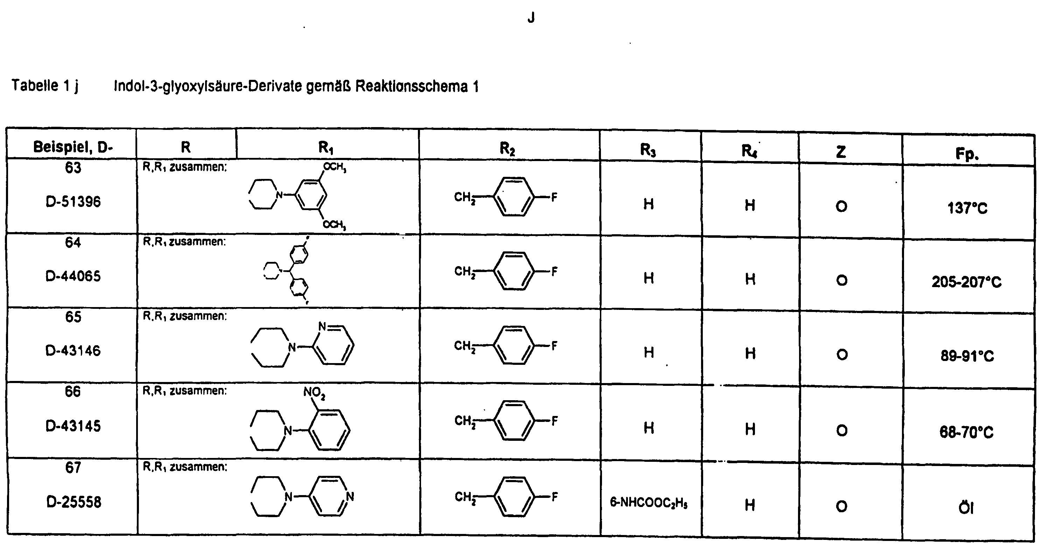

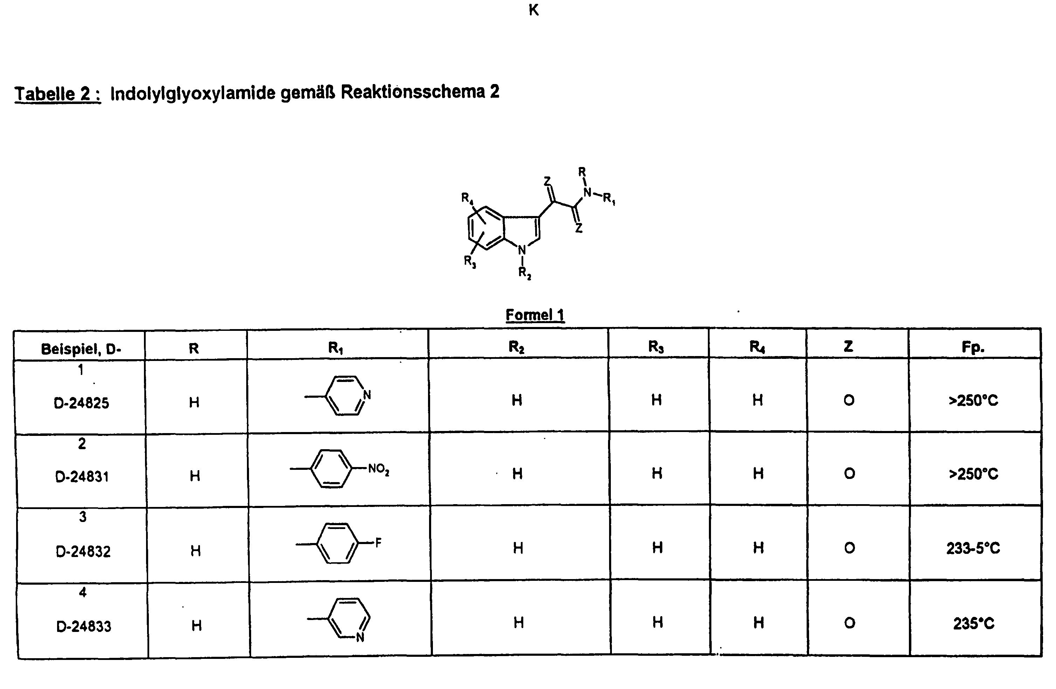

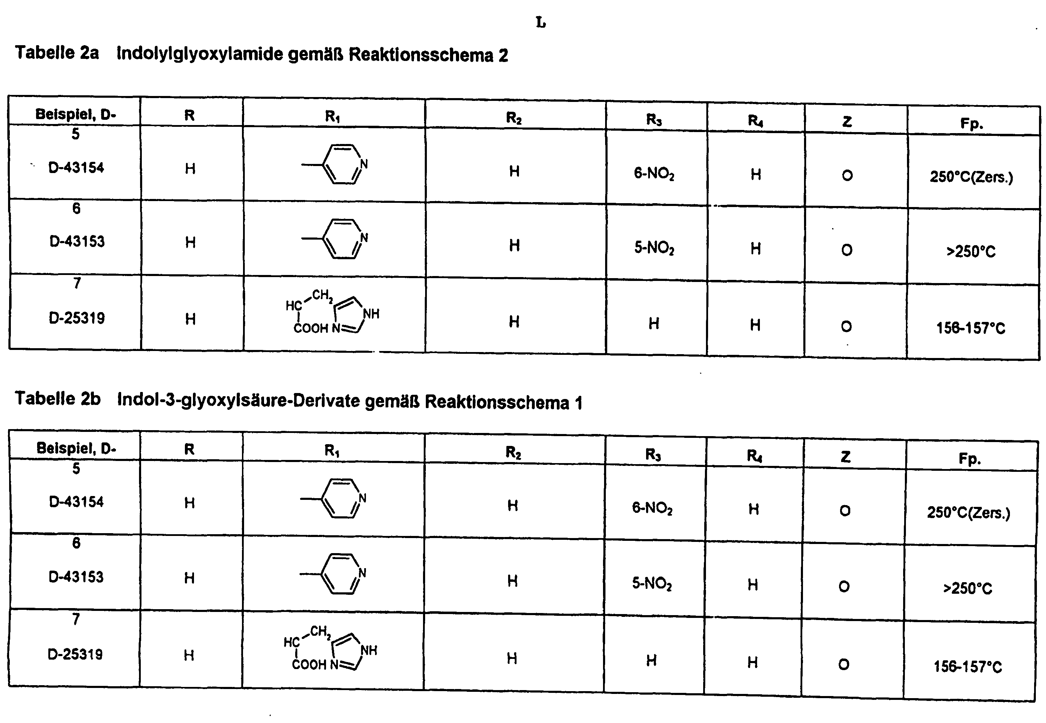

Bei den Untersuchungen mit der Tumorzellinie L1210, eine lymphatische Leukämie der Maus, zeigte sich nach intraparitonealer bzw. peroraler Gabe von D 24851 mit 100 und 147 mg/kg mehrfach-Gabe eine deutliche dosisabhängige Verlängerung der Überlebenszeit (Figur 1a und Figur 1b).

Aufgrund der guten therapeutischen Breite, die experimentell nachgewiesen wurde, kann die Wirksubstanz höher als handelsübliche Tumorpharmaka dosiert werden.

Ohne mit der nach folgenden Angabe den Umfang der Erfindung einzuschränken zu wollen ist zu sagen, daß Dosierungen ab etwa 20 mg bis zu 500 mg täglich oral möglich sind.
Bei intravenöser Gabe als Injektion oder als Infusion können bis zu 250 mg/Tag oder mehr je nach Körpergewicht des Patienten und individueller Verträglichkeit verabreicht werden.

**Tabelle 3**

| **Zusammenstellung D-24851 gemäß Beispiel 13** | | | | | | | |
|---|---|---|---|---|---|---|---|
| **D-24851 N-(Pyridin-4-yl)-[1-(4-chlorbenzyl)-indol-3-yl]-glyoxylamid** | | | | | | | |
| **Modell** | **Result.** | **SK-OV-3** | **KB** | **L1210** | **LNCaP** | **MCF-7** | **Tox** |
| XTT (µg/ml) | EC₅₀ | ≈ 0.03 | ≈ 0.017 | ≈ 0.017 | ≈ 0.03 | | |
| 1x ip (mg/kg) | DL₅₀ | | | | | | = 1000 |
| 1 x per os (mg/kg) | DL₅₀ | | | | | | > 1000 |
| Hohlfaser intraperitoneal 4 x 46 mg/kg ip | % INH | | keine Wirkung | 56 | | 38 | |
| Hohlfaser intraperitoneal 4 x 147 mglkg ip | % INH | | 12 | 60 | | 68 | |
| Hohlfaser subcutan 4 x 46 mg/kg ip | % INH | | 44 | keine Wirkung | | 47 | |
| Hohlfaser subcutan 4 x 147 mg/kg ip | % INH | | 35 | 67 | | 68 | |
| In Vivo: | | | | | | | |
| 1 x 681 mg/kg ip | % ILS | | | 0 | | | |
| 1 x 464 mg/kg ip | | | | 18 | | | |
| 4 x 215 mg/kg ip | % ILS | | | 13 | | | |
| 4 x 147 mg/kg ip | | | | 94 | | | |
| 7 x 100 mg/kg ip | % ILS | | | 35 | | | |
| 7 x 147 mg/kg ip | | | | 59 | | | |
| 1 x 681 mg/kg po | % ILS | | | 22 | | | |
| 4 x 215 mg/kg po | | | | 31 | | | |
| 7 x 100 mg/kg po | | | | 63 | | | |
| 7 x147 mg/kg po | | | | 75 | | | |
| 7 x 46 mg/kg ip | % WHI | | 33 | | | | |
| 2 x 215 mg/kg po | | | 18 | | | | |

**Tabelle 3a**

| **Substanz gemäß Beispiel** | **Tumorzellen XTT** | | | |
|---|---|---|---|---|
| **(D-Nummer)** | **KB** | **L 1210** | **LNCAP** | **SK-OV-3** |
| | EC₅₀ [µg/ml] | EC₅₀ [µg/ml] | EC₅₀ [µg/ml] | EC₅₀ [µg/ml] |
| 1 (D 24241) | 0,020 | 0,170 | >31,600 | 0,170 |
| 3 (D 24834) | 1,75 | 1,75 | 9,250 | 1,750 |
| 4 (D 24835) | 17,5 | 1,750 | >31,6 | 9,200 |
| 6 (D 24840) | 3,100 | 1,750 | >31,6 | 17,5 |
| 9 (D 24843) | 0,050 | 0,090 | 3,240 | 1,750 |
| 10 (D 24848) | 4,060 | 1,75 | >31,6 | 7,220 |
| 11 (D 24849) | 4,590 | 1,750 | 17,500 | 4,250 |
| 12 (D 24850) | >31,6 | 0,017 | >31,6 | >31,6 |
| 13 (D 24851) | 0,017 | 0,017 | 0,030 | 0,030 |
| 14 (D 24852) | 1,75 | 1,75 | 17,5 | 2,58 |
| 15 (D 24853) | >31,6 | 3,1 | >31,6 | >31,6 |
| 16 (D 24847) | 4,59 | 1,75 | 17,500 | 4,250 |
| Tabelle 2 (D 24831) | 17,5 | 17,5 | 17,5 | 17,5 |

### Weitere tierexperimentelle Ergebnisse:

Am Dunning Tumor konnte nach Gabe von 7x 100 mg/kg und 7x 147 mg/kg p.o. D-24851 ein Tumorwachstumsstop, bei einigen Tieren sogar eine Tumorregression beobachtet werden.

Die Testung der kristallinen Form brachte im Vergleich zur ursprünglichen Form keine Unterschiede.

### D-24851 verursacht keine DNA-Fragmentierung

Im Haematopoese Versuch wurde keiner der untersuchten Blutparameter durch die intraperitoneale Gabe von D-24851 verändert.

**Tabelle 4**

| **D 24241 N-(Pyridin-4-yl)-[1-(4-fluorbenzyl)-indol-3-yl] glyoxylamid gemäß Beispiel 1** | | | | | | | |
|---|---|---|---|---|---|---|---|
| **Modell** | **Result.** | **SK-OV-3** | **KB** | **L1210** | **LNCaP** | **MCF-7** | **Tox** |
| XTT (µg/ml) | EC₅₀ | ≈0.17 | ≈0.02 | ≈0.17 | >31.6 | | |
| 1x ip (mg/kg) | DL₅₀ | | | | | | ≈ 158 |
| 1x per os (mg/kg) | DL₅₀ | | | | | | > 1000 |
| Hohlfaser intraperitoneal 4 x 15.8 mg/kg ip | % INH | | 46 | 43 | | keine Wirkung | |
| Hohlfaser subcutan 4 x 15.8 mg/kg ip | % INH | | 81 | 68 | | 33 | |
| In Vivo: | | | | | | | |
| 1 x 14.7 mg/kg ip | % ILS | | | keine Wirkung | | | |
| 1 x 30 mg/kg per os | % ILS | | | keine Wirkung | | | |
| 1 x 464 mg/kg per os | % ILS | | | 44 | | | |
| 4 x 30 mg/kg per os | % ILS | | | keine Wirkung | | | |
| 6 x 30 mg/kg per os | % ILS | | | keine Wirkung | | | |
| 14 x 30 mgikg per os | % ILS | | | keine Wirkung | | | |
| 19 x 50 mg/kg per os | % ILS | | | 50 | | | |
| 2 x 46.4 mg/kg ip | %WHI | | 22 | | | | |
| 4 x 21.5 mg/kg ip | %WHI | | keine Wirkung | | | | |
| 2 x 215 mg/kg po | %WHI | | 47 | | | | |

**Tabelle 5**

| **Vergleich der Antitumorwirkung von D-24851 bzw. D-24241 mit Standardverbindungen** | | | |
|---|---|---|---|
| **Substanz** | **Tox. mg/kg** | **L1210 mg/kg** | **XTT EC 50 (µg/ml)** |
| **D-24851** | ≈1000 i.p. | 4x 147 i.p. | KB ≈ 0.017 |
| | | 94% ILS | L1210 ≈ 0.017 |
| | | | SKOV3 ≈ 0.03 |
| | | | LNCAP ≈ 0.03 |
| | | | |
| **D-24241** | ≈ 158 i.p. | 19x 50 p.o. | KB ≈ 0.02 |
| | | 50% ILS | L1210 ≈ 0.17 |
| | | | SKOV3 ≈ 0.17 |
| | | | LNCAP > 31.6 |
| | | | |
| Mitoxantron | 16 i.v. | 1x4.64 i.v. | KB ~0.174 |
| | | 144% ILS | L1210 < 0.0003 |
| | | | SKOV3 ~0.174 |
| | | | LNCAP ~0.017 |
| | | | |
| 5-Fluoruracil | ----- | 1 x 147 i.p. | ------ |
| | | 72% ILS | |
| | | 4x68.1 i. p. | |
| | | 83% ILS | |
| | | | |
| Methotrexat | ----- | 1 x 53.7 i.p. | KB - 0.007 |
| | | 39% ILS | L1210 n.d. |
| | | | SKOV3 > 31.6 |
| | | | LNCAP n.d. |
| Etoposid | ≈158.0 i.p | 1x 46.4i.p. | |
| | > 68.1 i.v. | 56% ILS | |
| | | | |
| Ratjadon | ~16.0 i.p. | 1x1.47 i.p. | KB < 0.003 |
| | ~30.0 i.v. | 22% ILS | L1210 < 0.003 |
| | | | SKOV3<0.003 |
| | | | LNCAP<0.003 |
| | | | |
| Epothilon B | ≈100.0 i.p. | 1x 10 i.p. | KB ~0.0002 |
| | | 44% ILS | L1210~0.0017 |
| | | | SKOV3~0.0031 |
| | | | LNCAP~0.014 |
| | | | |
| Taxol | ≈158 i.p. | 1x 14.7 i.v. | KB < 0.003 |
| | | 22% ILS | L1210 < 0.003 |
| | | 1x 46.4 i.v. | SKOV3< 0.003 |
| | | 61 % ILS | LNCAP< 0.003 |
| | | | |
| Vincristin | ≈ 3.0 i.v. | 1x1.0 i.p. | KB < 0.001 |
| | | 29% ILS | L1210 0.004 |
| | | | SKOV3 0.003 |
| | | | LNCAP 0.004 |
| | | | |
| Adriamycin | ≈ 27.0 i.v. | 1x14,7 i.v. | KB 0.15 |
| | | 111 % ILS | L1210 0.174 |
| | | | SKOV3 0.089 |
| | | | LNCAP 0.17 |
| | | | |
| Cisplatin | ≈ 16.0 i.p. | 1x3.16 i.p. | L1210 0.30 |
| | ≈ 73.0 p.o. | 38.9% ILS | |
| | | | |
| Crboplatin | ≈158.0 i.p. | 1x100 i.p. | ----- |
| | ≈841.0 p.o. | 41% ILS | |
| | | | |
| Lobaplatin | ≈ 34.0 i.p. | 1x14.7 i.p. | ----- |
| | | 55.0% ILS | |
| | | | |
| Cyclophosphamid | ≈340.7 i.v. | 1x46.4 i.v. | ----- |
| | | 40% ILS | |
| | | | |
| lfosfamid | ≈732 i.p. | 1x316 i.p. | ----- |
| | | 89% ILS | |
| | | | |
| Miltefosin | ≈ 46.4 i.p. | keine Wkg. | ----- |
| | ≈464-1000 p.o. | | |

## Patentansprüche

1. Verwendung von einem oder mehreren N-substituierten Indol-3-glyoxylamiden gemäß der allgemeinen Formel 1, 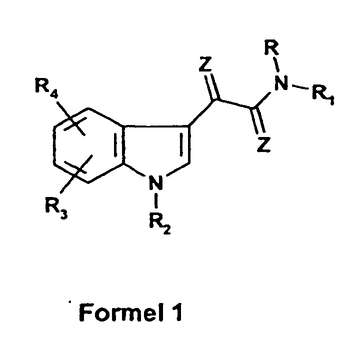 wobei die Reste R, R₁, R₂, R₃, R₄ und Z folgende Bedeutungen haben:
R = Wasserstoff
R₁ = 4-Pyridyl, 4-Fluorophenyl
R₂ = Benzyl, 4-Chlorbenzyl, 4-Fluorbenzyl, 3-Pyridylmethyl, 4-Brombenzyl
R₃ und R₄ = Wasserstoff und
Z = Sauerstoff,
sowie deren physiologisch verträgliche Säureadditionssalze bzw. sofern möglich deren N-Oxide, zur Herstellung eines Arzneimittels zur Behandlung von Tumorerkrankungen.

2. Verwendung von einem oder mehreren N-substituierten Indol-3-glyoxylamiden der allgemeinen Formel 1 nach Anspruch 1 zur Herstellung eines Arzneimittels zur Behandlung von Tumorerkrankungen, wobei das physiologisch verträgliche Säureadditionssalz aus der Gruppe bestehend aus Salzsäure, Schwefeläure, Phosphorsäure, Essigsäure, Milchsäure, Malonsäure, Maleinsäure, Fumarsäure, Gluconsäure, Glucuronsäure, Zitronensäure, Embonsäure, Methansulfonsäure, Trifluoressigsäure, Bemsteinsäure und 2-Hydroxyethansulfonsäure, ausgewählt ist.

3. Verwendung nach Anspruch 1 oder 2, wobei das N-substituierte Indol-3-glyoxylamid der allgemeinen Formel 1 ausgewählt ist aus:
D 24241 N-(Pyridin-4-yl)-[1-(4-fluorbenzyl)-indol-3-yl] glyoxylamid
D 24843 N-(Pyridin-4-yl)-(1-benzylindol-3-yl)-glyoxylamid
D 24850 N-(4-Fluorphenyl)-[1-(3-pyridylmethyl)-indol-3-yl]-glyoxylamid
D 24851 N-(Pyridin-4-yl)-[1-(4-chlorbenzyl)-indol-3-yl]-glyoxylamid oder
D-25505 N-(Pyridin-4-yl)-[1-(4-fluorbenzyl)-indol-3-yl] glyoxylamid HCl.

4. Verwendung von einem oder mehreren N -substituierten Indol-3-gloxylamiden gemäß der allgemeinen Formel 1 nach Anspruch 1 oder 2 sowie ggf. deren physiologisch verträglichen Säureadditionssalze bzw. sofem möglich deren N-Oxide, insbesondere jedoch eine oder mehrere Verbindungen gemäß Anspruch 3 sowie deren physiologisch verträgliche Säureadditionssalze bzw. sofem möglich deren N-Oxide, zur Herstellung eines Arzneimittels zur Behandlung von Tumorerkrankungen, durch perorale, parenterale, intravenöse, transdermale oder inhalative Applikation, wobei vorzugsweise bei oraler Gabe Dosierungen ab etwa.20 mg bis zu 500 mg täglich und bei intravenöser Gabe als Injektion oder als Infusion bis zu 250 mg/Tag oder mehr je nach Körpergewicht des Patienten und individueller Verträglichkeit verabreicht werden können.

5. Verwendung eines N -substituierten Indol-3-glyoxylamids der nachstehend genannten Formel:
D 24241 N-(Pyridin-4-yl)-[1-(4-fluorbenzyl)-indol-3-yl] glyoxylamid
sowie dessen physiologisch verträglichen Säureadditionssalze, wie beispielsweise dessen Hydrochlorid, bzw. sofern möglich deren N-Oxide, zur Herstellung eines Arzneimittels zur Behandlung von Tumorerkrankungen.

6. Verwendung eines N-substituierten Indol-3-glyoxylamids der nachstehend genannten Formel:
D 24843 N-(Pyridin-4-yl)-(1-benzylindol-3-yl)-glyoxylamid
sowie dessen physiologisch verträglichen Säureadditionssalze bzw. sofem möglich deren N-Oxide, zur Herstellung eines Arzneimittels zur Behandlung von Tumorerkrankungen.

7. Verwendung eines N-substituierten Indol-3-glyoxylamids der nachstehend genannten Formel:
D 24850 N-(4-Fluorphenyl)-[1-(3-pyridylmethyl)-indol-3-yl]-glyoxylamid
sowie dessen physiologisch verträglichen Säureadditionssalze bzw. sofem möglich deren N-Oxide, zur Herstellung eines Arzneimittels zur Behandlung von Tumorerkrankungen.

8. Verwendung eines N-substituierten Indol-3-glyoxylamids der nachstehend genannten Formel:
D 24851 N-(Pyridin-4-yt)-[1-(4-chtorbenzyt)-indot-3-yt]-gtyoxylamid
sowie dessen physiologisch verträglichen Säureadditionssalze bzw. sofern möglich deren N-Oxide, zur Herstellung eines Arzneimittels zur Behandlung von Tumorerkrankungen.

9. Verwendung von einem oder mehreren N-substituierten Indol-3-gloxylamiden gemäß der allgemeinen Formeln 1 nach Anspruch 1 sowie ggf. deren physiologisch verträglichen Säureadditionssalze und, sofern möglich, N-Oxide, insbesondere jedoch eine oder mehrere Verbindungen gemäß einem der vorhergehenden Ansprüche sowie deren physiologisch verträgliche Säureadditionssalze bzw. sofem möglich deren N-Oxide, zur Herstellung eines Arzneimittels zur Behandlung von Tumorerkrankungen, wobei das Arzneimittel einen pharmazeutisch verwendbaren Träger- und/oder Verdünnungs - bzw. Hilfsstoff aufweist und vorzugsweise in Form von Tabletten, Dragees, Kapseln, Lösungen zur Infusion oder Ampullen, Suppositorien, Pflaster, inhalativ einsetzbare Pulverzubereitungen, Suspensionen, Cremes und Salben vorliegt.

## Claims

1. Use of one or more N-substituted indole-3-glyoxylamides of the general formula 1 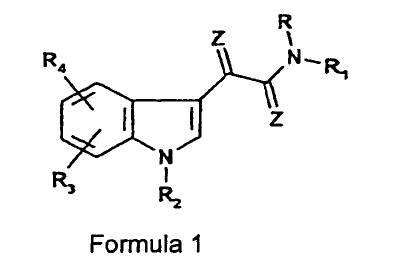 wherein the radicals R, R₁, R₂, R₃, R₄, N and Z have the following meanings:
R = hydrogen
R₁ = 4-pyridyl, 4-fluorophenyl
R₂ = benzyl, 4-chlorobenzyl, 4-fluorobenzyl, 3-pyridylmethyl, 4-bromobenzyl
R₃ and R₄ = hydrogen and
Z = oxygen
and their physiologically tolerable acid addition salts and, if possible, their N-oxides, for the preparation of a medicament for the treatment of tumor diseases.

2. The use of one or more N-substituted indole-3-glyoxylamides of the general formula 1 according to claim 1 for the preparation of a medicament for the treatment of tumor diseases, wherein the physiologically tolerable acid addition salt is selected from the group, consisting of hydrochloric acid, sulfuric acid, phosphoric acid, acetic acid, lactic acid, malonic acid, maleic acid, fumaric acid, gluconic acid, glucuronic acid, citric acid, embonic acid, methanesulfonic acid, trifluoroacetic acid, succinic acid and 2-hydroxyethansulfonic acid.

3. The use according to claim 1 or 2, wherein the N-substituted indole-3-glyoxylamide of the general formula 1 is selected from:
D 24241 N-(pyridine-4-yl)-[1-(4-fluorobenzyl)-indole-3-yl]glyoxylamide
D 24843 N-(pyridine-4-yl)-(1-benzylindole-3-yl)-glyoxylamide
D 24850 N-(4-fluorophenyl)-[1-(3-pyridylmethyl)-indole-3-yl]glyoxylamide
D 24851 N-(pyridine-4-yl)-[1-(4-chlorobenzyl)-indole-3-yl]glyoxylamide or
D 25505 N-(pyridine-4-yl)-[1-(4-fluorobenzyl)-indole-3-yl]glyoxylamide HCI.

4. The use of one or more N-substituted indole-3-glyoxylamides of the general Formula 1 according to claim 1 or 2 as well as optionally their physiologically tolerable acid addition salts and, if possible, their N-oxides, in particular however one or more compounds according to claim 3 and their physiologically tolerable acid addition salts and, if possible, their N-oxides, for the preparation of a medicament for treating tumor diseases by peroral, parenteral, intravenous, transdermal or inhalative application, wherein preferably by oral administration doses from about 20 mg up to about 500 mg daily and by intravenous administration as injection or as infusion up to 250 mg/day or more depending on the body weight of the patient and individual tolerance can be administered.

5. The use of a N-substituted indole-3-glyoxylamide of the Formula mentioned below:
D 24241 N-(pyridine-4-yl)-[1-(4-fluorobenzyl)-indole-3-yl]glyoxylamide
and its physiologically tolerable acid addition salts, such as for example its hydrochloride, and, if possible, its N-oxides for the preparation of a medicament for treatment of tumor diseases.

6. The use of a N-substituted indole-3-glyoxylamide of the Formula mentioned below:
D 24843 N-(pyridine-4-yl)-([1-benzylindole-3-yl)glyoxylamide
and its physiologically tolerable acid addition salts and, if possible, its N-oxides for the preparation of a medicament for treatment of tumor diseases.

7. The use of a N-substituted indole-3-glyoxylamide of the Formula mentioned below:
D 24850 N-(4-fluorophenyl)-[1-(3-pyridylmethyl)-indole-3-yl]glyoxylamide
and its physiologically tolerable acid addition salts and, if possible, its N-oxides for the preparation of a medicament for treatment of tumor diseases.

8. The use of a N-substituted indole-3-glyoxylamide of the Formula mentioned below:
D 24851 N-(pyridine-4-yl)-[1-(4-chlorobenzyl)-indole-3-yl]glyoxylamide
and its physiologically tolerable acid addition salts and, if possible, its N-oxides for the preparation of a medicament for treatment of tumor diseases.

9. The use of one or more N-substituted indole-3-glyoxylamides of the general Formula 1 according to claim 1 and optionally their physiologically tolerable acid addition salts and, if possible, N-oxides, particularly however one or more compounds according to one of the preceding claims as well as their physiologically tolerable acid addition salts and, if possible, N-oxides, for the preparation of a medicament for treatment of tumor diseases, wherein the medicament has a pharmaceutically utilizable excipient and/or diluent or auxiliary and is preferably in the form of tablets, coated tablets, capsules, solutions for infusion or ampoules, suppositories, plaster, inhalative usable powder preparations, suspensions, creams and ointments.

## Revendications

1. Utilisation d'un ou plusieurs indol-3-glyoxylamides N-substitués de formule générale 1 : 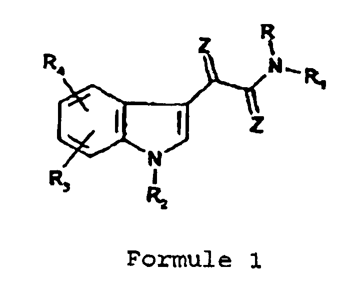 dans laquelle les radicaux R, R₁, R₂, R₃, R₄ et Z ont les significations suivantes:
R = hydrogène,
R₁ = 4-pyridyle, 4-fluorophényle,
R₂ = benzyle, 4-chlorobenzyle, 4-fluorobenzyle, 3-pyridylméthyle, 4-bromobenzyle,
R₃ et R₄ = hydrogène, et
Z = oxygène,
ainsi que de leurs sels d'addition d'acides physiologiquement compatibles ou, dans la mesure du possible, de leurs N-oxydes, pour préparer un médicament pour le traitement de maladies tumorales.

2. Utilisation d'un ou plusieurs indol-3-glyoxylamides N-substitués de formule générale 1 selon la revendication 1, pour préparer un médicament pour le traitement de maladies tumorales, dans laquelle le sel d'addition d'acide physiologiquement compatible est choisi dans le groupe constitué de l'acide chlorhydrique, de l'acide sulfurique, de l'acide phosphorique, de l'acide acétique, de l'acide lactique, de l'acide malonique, de l'acide maléique, de l'acide fumarique, de l'acide gluconique, de l'acide glucuronique, de l'acide citrique, de l'acide embonique, de l'acide méthanesulfonique, de l'acide trifluoracétique, de l'acide succinique et de l'acide 2-hydroxyéthanesulfonique.

3. Utilisation selon la revendication 1 ou 2, dans laquelle l'indol-3-glyoxylamide N-substitué de formule générale 1 est choisi parmi :
D 24241 N-(pyridin-4-yl)-[1-(4-fluorobenzyl)-indol-3-yl]glyoxylamide,
D 24843 N-(pyridin-4-yl)-(1-benzylindol-3-yl)-glyoxylamide,
D 24850 N-(4-fluorophényl)-[1-(3-pyridylrnéthyl)-indol-3-yl]-glyoxylamide,
D 24851 N-(pyridin-4-yl)-[1-(4-chlorobenzyl)-indol-3-yl]glyoxylamide ou de
D 25505 N-(pyridin-4-yl)-[1-(4-fluorobenzyl)-indol-3-yl]glyoxylamide, HCl.

4. Utilisation d'un ou plusieurs indol-3-glyoxylamides N-substitués de formule générale 1 selon la revendication 1 ou 2 ainsi qu'éventuellement de leurs sels d'addition d'acides physiologiquement compatibles ou, dans la mesure du possible, de leurs N-oxydes, mais en particulier d'un ou plusieurs composés selon la revendication 3 ainsi que de leurs sels d'addition d'acides physiologiquement compatibles ou, dans la mesure du possible, de leurs N-oxydes, pour préparer un médicament pour le traitement de maladies tumorales, par application orale, parentérale, intraveineuse, transcutanée ou par inhalation, dans laquelle on peut administrer, de préférence par voie orale, des doses de 20 mg environ jusclu'à 500 mg par jour et, par administration intraveineuse par injection ou par perfusion, jusqu'à 250 mg/jour ou plus en fonction du poids du patient et de la compatibilité individuelle.

5. Utilisation d'un indol-3-glyoxylamide N-substitué de formule ci-après :
D 24241 N-(pyridin-4-yl)-[1-(4-fluorobenzyl)-indol-3-yllglyoxylamide,
ainsi que de ses sels d'addition d'acides physiologiquement compatibles, tels que son chlorhydrate, ou dans la mesure du possible de ses N-oxydes, pour préparer un médicament pour le traitement de maladies tumorales.

6. Utilisation d'un indol-3-glyoxylamide N-substitué de formule ci-après :
D 24843 N-(pyridin-4-yl)-(1-benzylindol-3-yl)-glyoxylamide,
ainsi que de ses sels d'addition d'acides physiologiquement compatibles ou, dans la mesure du possible de ses N-oxydes, pour préparer un médicament pour le traitement de maladies tumorales.

7. Utilisation d'un indol-3-glyoxylamide N-substitué de formule ci-après :
D 24850 N-(4-fluorophënyl)-[1-(3-pyridylméthyl)-indo2-3-yl]-glyoxylamide,
ainsi que de ses sels d'addition d'acides physiologiquement compatibles ou, dans la mesure du possible de ses N-oxydes, pour préparer un médicament pour le traitement de maladies tumorales.

8. Utilisation d'un indol-3-glyoxylamide N-substitué de formule ci-après:
D 24851 N-(pyridin-4-yl)-[1-(4-chlorobenzyl)-indol-3-yl]-glyoxylamide,
ainsi que de ses sels d'addition d'acides physiologiquement compatibles ou, dans la mesure du possible de ses N-oxydes, pour préparer un médicament pour le traitement de maladies tumorales.

9. Utilisation d'un ou plusieurs indol-3-glyoxylamides de formule générale 1 selon la revendication 1, ainsi qu'éventuellement de leurs sels d'addition d'acides physiologiquement compatibles et, dans la mesure du possible de leurs N-oxydes, mais en particulier d'un ou plusieurs composés selon l'une quelconque des revendications précédentes ainsi que de leurs sels d'addition d'acides physiologiquement compatibles ou, dans la mesure du possible de leurs N-oxydes, pour préparer un médicament pour le traitement de maladies tumorales, le médicament présentant un véhicule et/ou un diluant ou un adjuvant utilisable pharmaceutiquement et qui se présente, de préférence, sous la forme de comprimés, de dragées, de capsules, de solutions pour perfusion ou d'ampoules, de suppositoires, de pansements, de préparations en poudre utilisables pour inhalation, de suspensions, de crèmes et de pommades.
